# EUROPEAN PATENT APPLICATION

(11) **EP 4 252 800 A1**
(43) Date of publication of application: **04.10.2023**
(21) Application number: 22165139.1
(22) Date of filing: 29.03.2022
(51) Int. Cl.: A61M 5/20, A61M 5/30, A61M 5/315, A61M 5/24, A61M 5/31

(54) **DEVICE FOR HIGH-SPEED SUBCUTANEOUS INOCULATION OF LIQUID PRODUCTS**

(71) Applicant: Dondi Ingegneria Srl, 20136 Milano (IT)
(72) Inventor: DONDI, Alessandro, 27010 Borgarello (PV) (IT); BONDIOLI, Luca, 20900 Monza (MB) (IT); CERETTI, Sacha, Milano (IT)
(74) Representative: Luppi Intellectual Property S.r.l.

(57) **Abstract**

A device (10) for high-speed subcutaneous inoculation of liquid products provides a handle (11) and a barrel (12) suitable for receiving an ampoule (A) containing the liquid product and provided with a plunger (B) and with a micro-nozzle (C) for ejecting the liquid product upon actuation of the plunger (B); in the barrel (12) a spring (20) is received acting on a hammer (22) suitable in turn for acting on the plunger (B); the spring (20) is loaded elastically, held and released so as to operate the hammer (22) and consequently the plunger (B) for ejecting the liquid product; the handle (11) is extendable and a lever-operated ratchet mechanism is provided which is lever-operated through the handle (11) for progressive loading of the spring (20); the extension of the handle (11) increases the lever arm of the handle (11) to operate the ratchet mechanism. This results in a reduced loading effort on the spring (20).

## Description

### Background of the invention

The present invention relates to a device able to inoculate subcutaneously at high-speed a liquid product, for the most varied uses in the medical sector.

### Prior art

In the medical field, to inject a liquid drug subcutaneously into a patient, the common syringe with needle is mainly used. However, the use of a needle is not without problems.

First, a significant part of the population suffers from a real needle phobia.

In addition, improper use of the syringe with needle can lead to side effects such as pain, hematoma, scar tissue formation, infections, or abscesses. These side effects are independent of the type of drug contained in the syringe and are exclusively related to the result of the injection.

To overcome these problems, the so-called syringe without needle has been proposed for a long time now, which allows the liquid drug to be inoculated without the use of a needle.

Basically, the syringe without a needle is a device which allows the liquid drug to be inoculated directly in the form of a very thin, high-speed jet which crosses the cutaneous surface layers. To this end, in the prior art, a type of device provides that on the device an ampoule containing the liquid drug be mounted, which has a micro-nozzle for ejecting the liquid drug, and is provided with a plunger, on which, through a hammer, a spring of the device loaded before use acts. In use, the device is placed on the skin surface with the micro-nozzle directed to the inoculation point, and thus the spring is released by means of a release button to move at high-speed the plunger and form through the micro-nozzle the very thin jet that quickly penetrates the skin.

However, such a device is not practical in use.

In fact, to be able to move the plunger at high-speed, the elastic constant of the spring has to be high, and this entails a considerable effort for loading the spring.

The spring can be loaded by the operator by acting directly on a loading mechanism of the device, but this requires a great effort that not all operators are able to exert.

The spring can be loaded using a special additional device with which the operator loads the spring without having to exert a great effort. Using an additional device, however, complicates the instrumentation necessary for inoculation and complicates the operator's activity. The cost of this instrumentation which includes two devices is also high.

### Object of the invention

The object of the present invention is to propose a device for high-speed subcutaneous inoculation of liquid products that does not have the limits in the practical use of the known inoculation devices seen above not using the needle.

### Brief description of the invention

This object is achieved by an inoculation device according to the first claim.

### Brief description of the drawings

To better understand the invention, a non-limiting exemplary embodiment thereof is described below, illustrated in the attached drawings in which:
Fig. 1 is a perspective view of an inoculation device according to the invention;
Fig. 2 is a side view of the device of Fig.1;
Fig. 3 is a perspective view of the device of Fig.1 with parts removed;
Fig. 4 is a section side view of the device of Fig.1;
Fig. 5 is a section and exploded side view of the device of Fig.1;
Fig. 6 is a rear section view of the device of Fig.1;
Fig. 7 is a perspective exploded view of the device of Fig.1;
Fig. 8 is another perspective exploded view of the device of Fig.1;
Fig. 9 is a further perspective exploded view of the device of Fig.1;
Figs 10,11,12 are respectively a perspective exploded view, an exploded side view and another perspective view of a detail of the device of Fig.1;
Figs 13,14 are two side views of the device of Fig.1 as illustrated in Fig.4, in two different operating positions.

### Detailed description of the invention

The illustrated device, generically indicated by 10, is suitable for inoculating subcutaneously at high-speed a liquid product contained in an ampoule A provided with a plunger B and with a dispensing micro-nozzle C, for the most varied uses in the medical field.

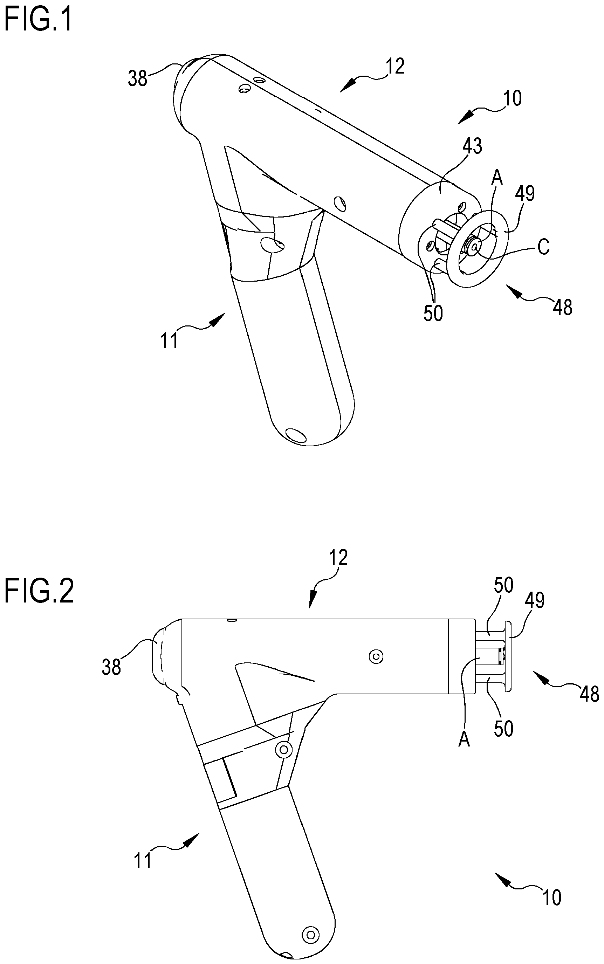

The device 10, as well clear from Figs 1,2, has a pistol-like shape and provides a handle 11 and a barrel 12.

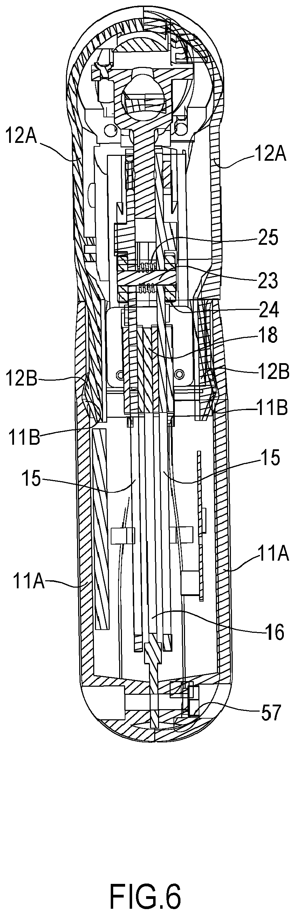

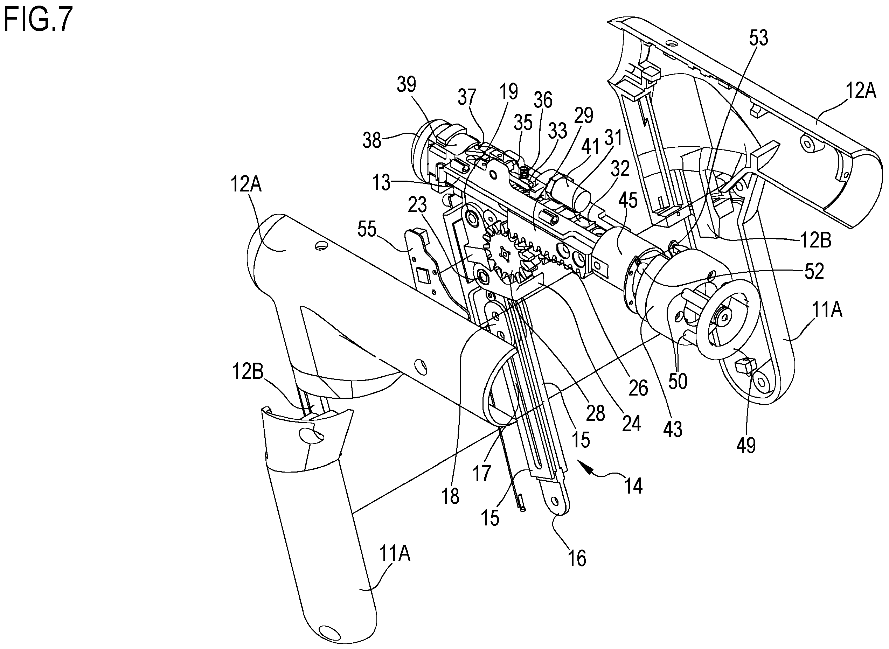

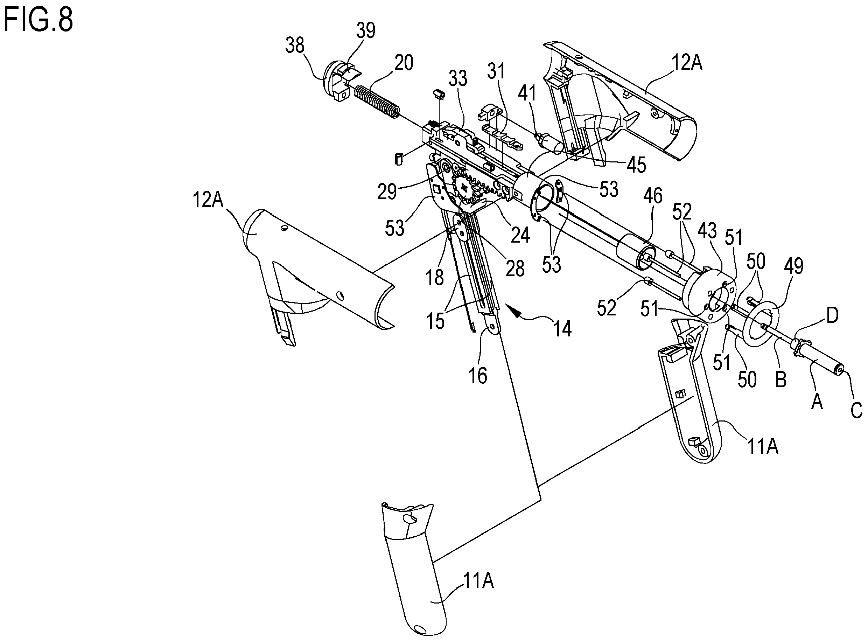

As shown in Figs 6,7,8, the handle 11 is formed by two half-shells 11A fixed together and the barrel 12 is also formed by two half-shells 12A fixed together.

The half-shells 11A and 12A form a box-like body of the device 10.

With particular reference to Fig.6, the handle 11 is snap-coupled with the barrel 12 by means of two recesses 11B, each formed inside a respective half-shell 11A of the handle 11, into which two respective elastic hooks 12B are inserted, each obtained inside a respective half-shell 12A of the barrel 12.

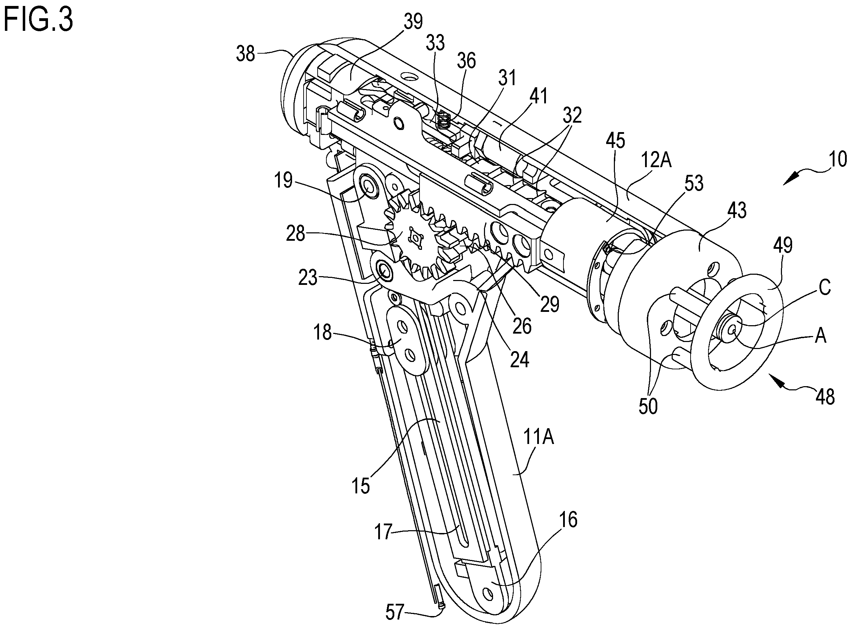

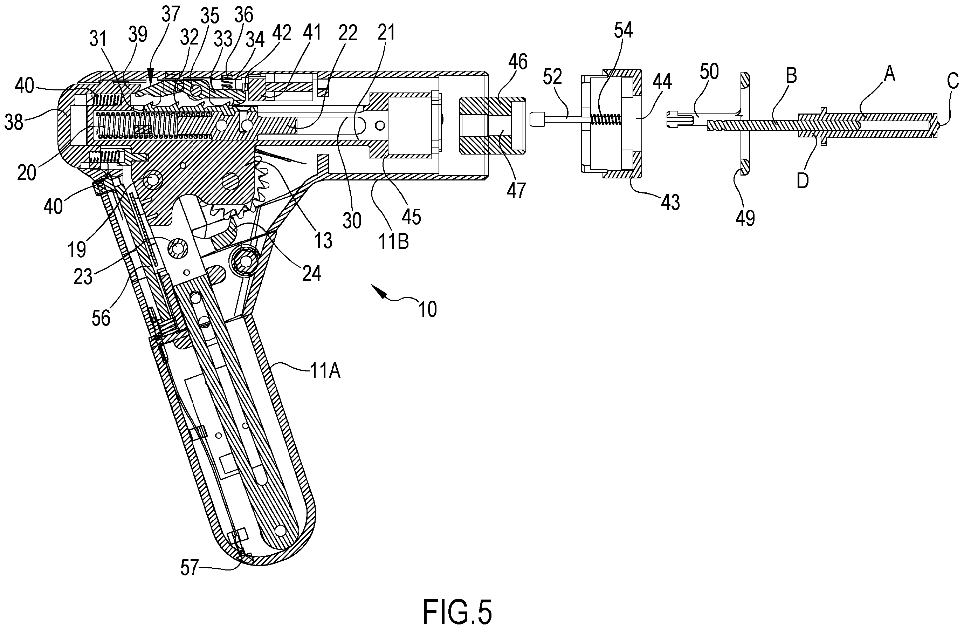

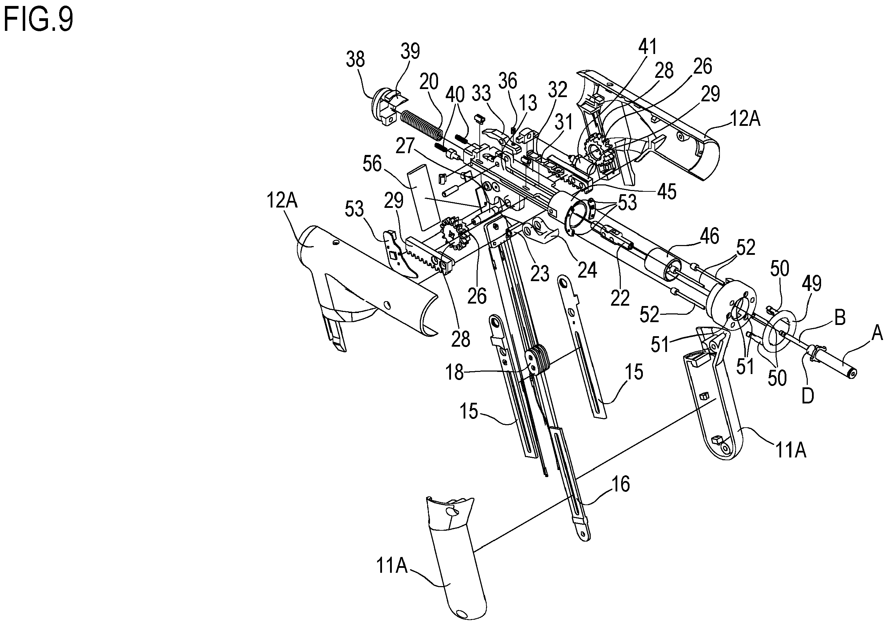

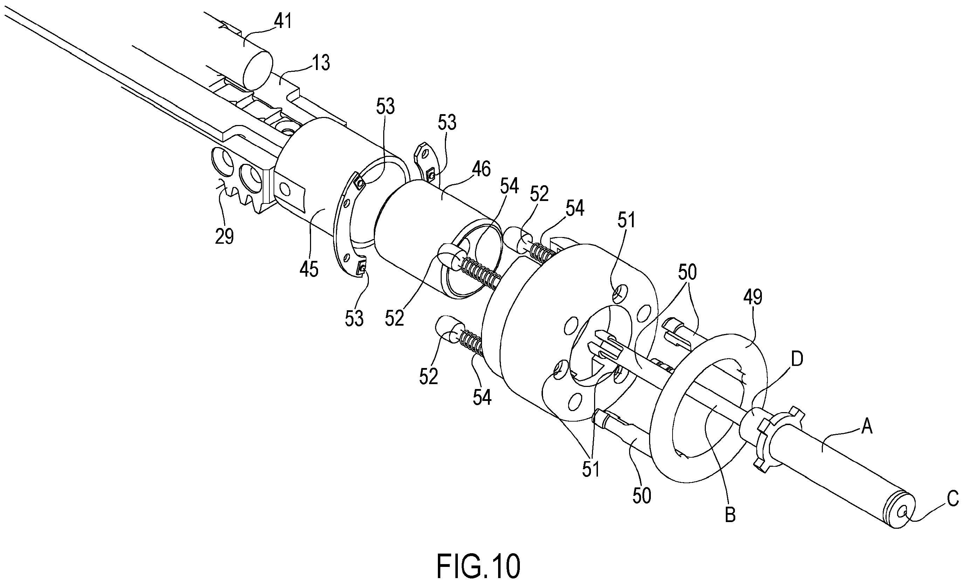

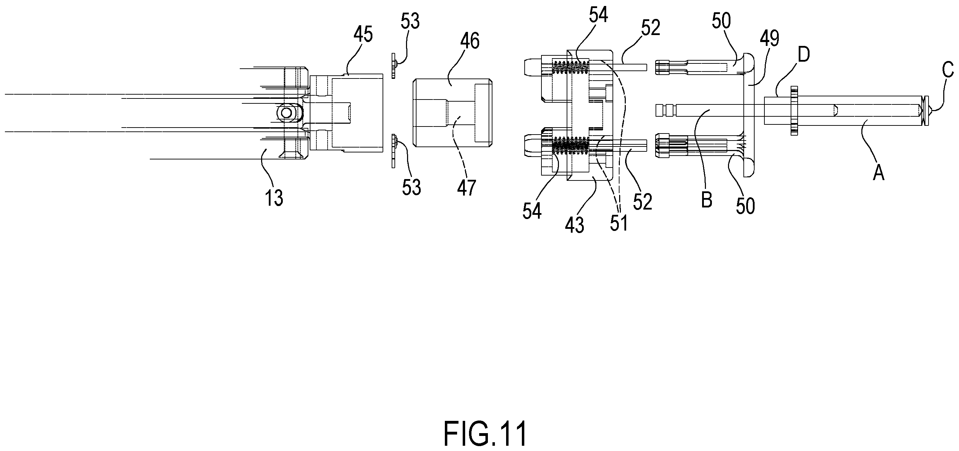

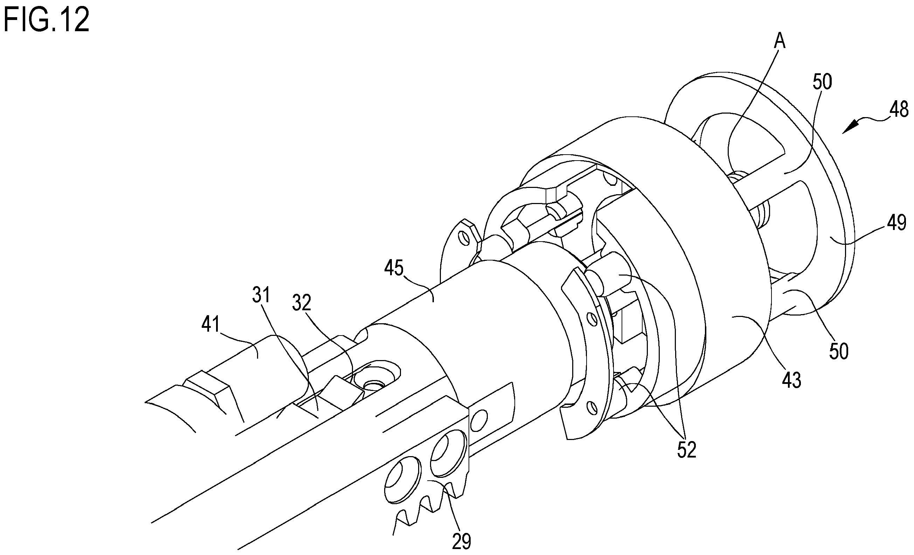

With reference to Figs 3-12, the components inside the box-like body of the device 10 are described below.

Inside the box-like body, there is a support frame 13 fixed to the barrel 12, to which the components of the device 10 are connected.

At the handle 11, an extendable lever 14 is provided. The lever 14 is formed by a pair of parallel rods 15 spaced apart and fixed to each other and by a third middle rod 16 frictionally sliding between the two rods 15. For sliding, the two rods 15 are provided with respective opposite guide slots 17, along which a slider 18 integral with the rod 16 is mounted in a frictionally sliding manner. The two rods 15 are hinged by means of a pin 19 to the frame 13. The middle rod 16 is integral with the handle 11.

The extendable lever 14 is intended for loading a drive spring 20 with a high elastic constant, received in a seat 21 of the frame 13 inside the barrel 12. The spring 20 pushes on a hammer 22 which, once the spring 20 is released, is intended to act on the plunger B of the ampoule A for high-speed subcutaneous inoculation of the liquid product.

The following ratchet mechanism is provided for loading the spring 20.

To the pair of rods 15 a beak element 24 is hinged on which acts a torsion spring 25 visible in Fig.6. On the action of the spring 25, the beak element 24 engages with a pair of opposite and coaxial shark-toothed gear 26, pivoted by means of a pin 27 to the frame 13. Another pair of straight-toothed gears 28, arranged externally to the gears 26 is coupled to the pair of gears 26 so that each gear 28 is arranged alongside and integral with a respective gear 26. The pair of gears 28 engages with a pair of opposite racks 29 sliding along guide slots 30 of the frame 13 and integral with the hammer 22 on which the spring 20 urges.

To hold the spring 20 and release it once the spring 20 has been loaded, the following hooking/unhooking mechanism is provided.

A side bar 31 provided with a series of slanted teeth 32 (in the example four in number) is integral with the pair of racks 29. A beaked end 34 of a rotating hook 33 engages one or the other of the teeth 32 of the toothed side bar 31; the hook 33 is hinged at an intermediate point, by means of a pin 35, to the frame 13; a spring 36 acts on the hook 33 to maintain the engagement with the toothed bar 31. At the end opposite the beaked end 34, the hook 33 has a tilted plane 37 on which an external button 38 acts with a lug part 39 thereof opposite to two springs 40, to determine an unhooking rotation of the hook 33. The button 38 is mounted at the rear end of the barrel 12 so as to protrude slightly from the latter.

A locking/unlocking mechanism is also mounted on the frame 13, providing an electromagnetic actuator 41 which actuates a locking/unlocking pin 42 between a retracted position and an extracted position in which the pin 42 keeps the hook 33 engaged with the tooth 32 of the side bar 31.

A hollow inoculation head 43 having a through hole 44 aligned with the spring 20 and the hammer 22 is fixed to the end of the barrel 12 opposite to the external button 38. At the head 43, a hollow cylindrical element 45 is fixed to the frame 13 inside which a cylindrical connection 46 is received with a through hole including a threaded hole 47 in which a correspondingly threaded part D of the ampoule A is intended to be screwed; the threaded hole 47 is aligned with the spring 20 and the hammer 22.

A contact sensor 48 is mounted on the head 43, allowing the device 10 to be placed correctly and with the appropriate pressure on the skin surface through which the liquid product has to be inoculated. The contact sensor 48 provides a contact ring 49 from which extend, parallel to the axis of the ring and in an angularly equidistant way, three stems 50 sliding in three corresponding through holes 51 of the head 43; the three sliding stems 50 are connected to three rod-shaped pressers 52 intended to come into contact with three corresponding microswitches 53 mounted on the cylindrical element 45. Three corresponding springs 54, received in the head 43, act on the three stems 50, pushing the stems 50 elastically outwards, i.e. to the extracted position.

The electromagnetic actuator 41 and the microswitches 53 are electrically connected to an electronic command and control board 55, fixed to the frame 13 in the connection area between the handle 11 and the barrel 12.

The device 10 is also provided with a rechargeable battery 56, placed near the electronic board 55 and electrically connected to the latter. For recharging the battery 56, electric contacts 57 are provided on the bottom of the handle 11, intended to couple with corresponding electric contacts of a recharging base, not shown, in which the handle 11 is inserted.

The electronic board 55 manages the operation of the electromagnetic actuator 41 according to the state of the microswitches 53 and manages the recharging of the battery 56.

The operation of the device 10 is as follows, with reference to the operations to be performed by the operator.

First, the ampoule A is prepared with the liquid product already inserted and consequently the plunger B extracted.

The drive spring 20 is then loaded by means of the ratchet mechanism disclosed above.

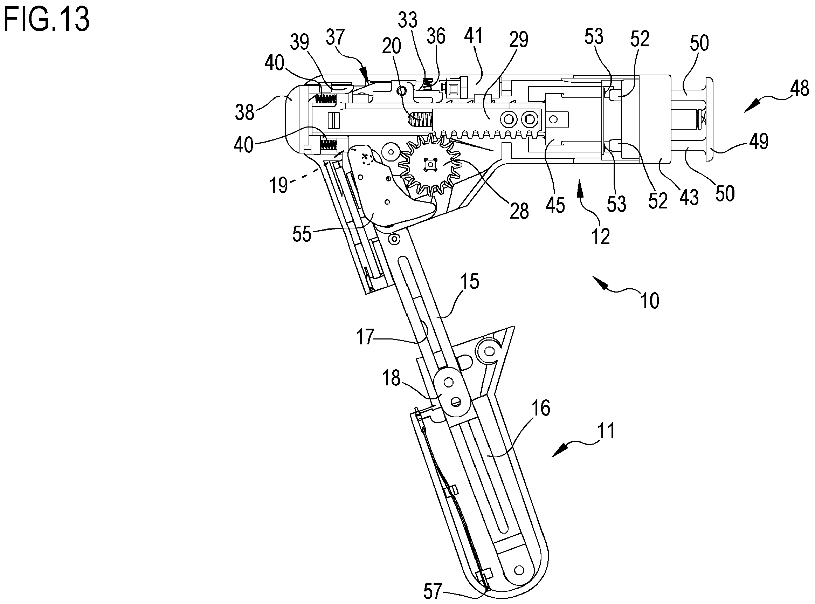

To this end, with reference to Fig.13, the handle 11 is extracted in a position away with respect to the barrel 12. This extraction is possible thanks to the fact that the handle 11 is constrained on the middle rod 16 which in turn can slide along the pair of rods 15 through the cursor 18. During extraction, the force of snap coupling between the handle 11 and barrel 12 has to be overcome.

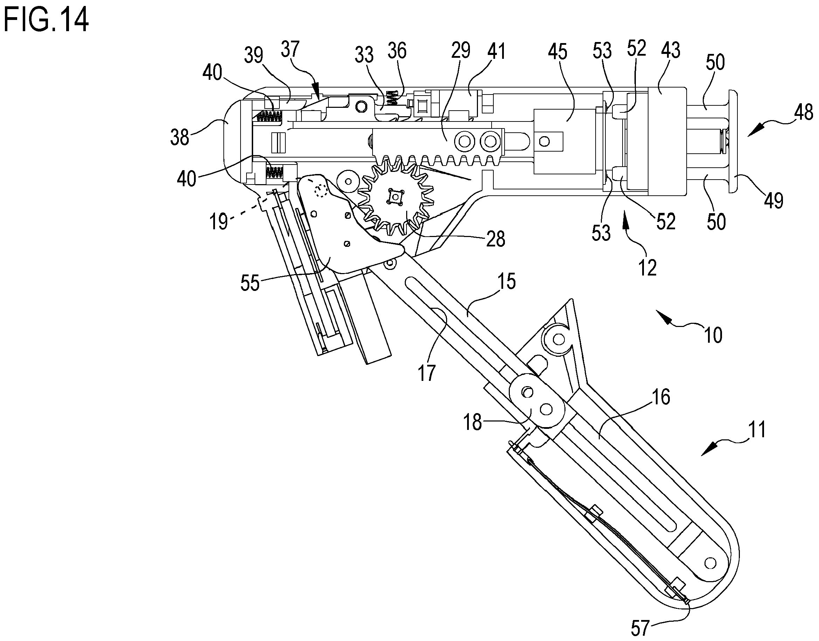

For a first loading action, the handle 11 is rotated towards the barrel 12, as shown in Fig.14, and then rotated in the opposite direction to the initial position. In this way, the beak element 24 rotates in one direction the gears 26 and therefore the coaxial gears 28 which in turn linearly move the racks 29 along the guides 30 in a direction such as to determine a first loading position of the spring 20. The first tooth 32 of the side bar 31, integral with the racks 29, which the hook 33 meets, locks the side bar 31 and therefore the spring 20 and the hammer 22 in this first loading position.

It is proceeded in this way with a series of loading actions equal to the number of teeth 32 of the side bar 31 until the hook 33 engages the last tooth 32 of the side bar 31.

Once the loading of the spring 20 has been terminated, the handle 11 is returned to the coupling position with the barrel 12 by a sliding closure in a direction opposite to extraction.

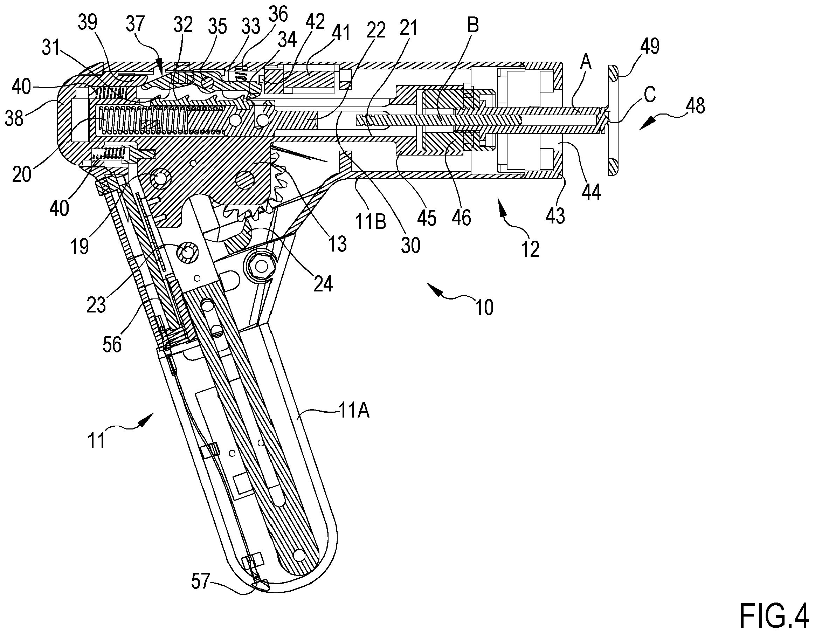

The threaded part D of the ampoule A is then screwed in the threaded hole 47 of the cylindrical connection 46. When the screwing has been completed, the plunger B is located at the hammer 22 at a certain distance therefrom, as shown in Fig.4.

At this point, holding the device 10 by the handle 11, the head 43 with the contact sensor 48 is placed on the skin surface of the patient through which the product has to be inoculated.

When placing the head 43 with the contact sensor 48, the three stems 50 slide in the holes 51 towards the inside of the device 10 in contrast to the action of the springs 54. If the ring 49 of the contact sensor 48 contacts correctly the skin surface and the pressure exerted on the skin surface is appropriate, the three pressers 52 press all three microswitches 53, which command the electronic board 55 to activate the electromagnetic actuator 41. The activation of the electromagnetic actuator 41 involves the retraction of the pin 42 and therefore the release of the hook 33.

The device 10 is now in the condition of being able to operate. By pressing the outer button 38 in contrast to the springs 40, the lug 39 of the outer button 38 goes to act on the tilted plane 37 of the hook 33 causing a disengagement rotation thereof from the last tooth 32. Consequently, the spring 20 is released and exerts a strong thrust on the hammer 22, which in turn, after a short stroke, comes into contact with the plunger B and pushes it with equal force inside the ampoule A, determining the liquid product to come out from the micro-nozzle C in the form of a very thin, high-speed jet which penetrates subcutaneously.

If the ring 49 of the contact sensor 48 is not correctly placed on the skin surface and/or the pressure exerted on the skin surface is not appropriate, all three microswitches 53 will not be pressed by the three pressers 52 and therefore the hook 33 will not be unlocked and the device 10 cannot operate.

The device 10 described and illustrated has various advantages.

The loading of the spring 20 is carried out without great effort thanks to the extendable lever 14 and to the ratchet mechanism seen above. In fact, the high lever arm obtained with the extendable lever 14 and the progressive loading obtainable with the ratchet mechanism allow the operator to load the spring 20 without great effort.

The contact sensor 48 ensures the correct inoculation of the product as it allows the operator to operate only when the device 10 is so positioned and pressed on the skin surface so that the very thin jet can pass through the skin surface.

The pistol-like shape gives to the device 10 compactness and allows it to be handled comfortably and instinctively.

The device 10 is, amongst other things, not too complex and therefore of a not high cost.

Luminous and/or acoustic lights can be mounted on the device 10 to signal the operating condition of the device 10 and the charge status of the battery 56.

Variations in the configuration and in the number of the internal components of the device with respect to what has been described and illustrated can be envisaged.

The microswitch contact sensor described and illustrated is simple and effective. However, the use of other types of contact sensors, for example magnetic sensors, cannot be ruled out.

## Claims

1. Device (10) for high-speed subcutaneous inoculation of liquid products, comprising a handle (11) and comprising a barrel (12) suitable for receiving an ampoule (A) containing the liquid product and provided with a plunger (B) and with a micro-nozzle (C) for ejecting the liquid product upon actuation of the plunger (B), wherein in the barrel (12) there are slidingly received a spring (20) and a hammer (22) on which the spring (20) acts and which is in turn suitable for acting on the plunger (B), wherein the spring (20) is loaded elastically, is held, and is released so as to operate the hammer (22) and consequently the plunger (B) for ejecting the liquid product, wherein the handle (11) is extendable and a ratchet mechanism (23-30) is provided which is lever-operated through the handle (11) for progressive loading of the spring (20), the extension of the handle (20) allowing the lever arm of the handle (11) to be increased to operate the ratchet mechanism (23-30).

2. Device according to claim 1, wherein a hooking/unhooking mechanism (31-40) is provided for holding the spring (20) once loaded and for releasing the spring (20), wherein the barrel (12) is provided at one end with an inoculation head (43), at which the ampoule (A) is suitable for being fixed, and wherein the inoculation head (43) is provided with a contact sensor (48) connected to a locking/unlocking mechanism (41,42) of the hooking/unhooking mechanism (31-40) to authorize the release or not of the spring (20) according to the contact and pressure conditions of the inoculation head (43) on the skin surface.

3. Device according to claim 1 or 2, wherein, for extending the handle (11), there is an extendable lever (14) hinged to a fixed part (13) of the device and integral with the handle (11).

4. Device according to claim 3, wherein the extendable lever (14) comprises a pair of spaced apart parallel rods (15), fixed each other, and hinged to the fixed part (13) of the device, and a third middle rod (16), frictionally sliding between the pair of rods (15) and integral with the handle (11).

5. Device according to claim 3 or 4, wherein the ratchet mechanism comprises a beak element (24), hinged to the extendable lever (14), which engages elastically with one or more shark-toothed gears (26), pivoted to the fixed part (13) of the device and rotating integrally with one or more straight-toothed gears (28) which engage one or more racks (29) sliding along the fixed part (13) of the device and integral with the hammer (22).

6. Device according to claim 2, wherein the hooking/unhooking mechanism comprises a bar (31) provided with a series of teeth (32) and integral with the hammer (22), a rotating hook (33) which elastically engages with the one or other of the teeth of the bar (31), and an external button (38) suitable to act with elastic contrast on the hook (33) to determine an unhooking rotation of the hook (33).

7. Device according to claim 6, wherein the button (38) is mounted at the end of the barrel (12) opposite to the end in which the inoculation head (43) is arranged.

8. Device according to claim 6 or 7, wherein the locking/unlocking mechanism comprises an electromagnetic actuator (41) which operates a locking pin (42) between a retracted position and an extracted position in which the pin (42) keeps the hook (33) engaged with the tooth (32) of the bar (31).

9. Device according to claim 2, wherein the contact sensor (48) comprises a contact ring (49) from which one or more pressing elements (52) sliding in the inoculation head (43) extend, which act with elastic contrast on one or more corresponding microswitches (53) which enable or disable the locking/unlocking mechanism (41, 42) according to the contact and pressure conditions of the contact ring (49) on the skin surface.

10. Device according to claim 9, wherein the locking/unlocking mechanism (41,42) is of electromagnetic type and wherein an electronic command and control board is provided, which is electrically connected to the locking/unlocking mechanism (41,42) and to said one or more microswitches (53).

11. Device according to claim 10, wherein a rechargeable battery (56) is provided for powering the electric components.

12. Device according to any one of the preceding claims, having a substantially pistol-like shape.
